# FASCICULE DE BREVET EUROPEEN

(11) **EP 1 435 904 B1**
(45) Date de publication et mention de la délivrance du brevet: **30.03.2011**
(21) Numéro de dépôt: 02800620.3
(22) Date de dépôt: 30.09.2002
(51) Int. Cl.: C11D 1/66, A61K 8/06, A61K 8/34, A61K 8/60, A61Q 19/00

(54) **UTILISATION D'ALKYLPOLYXYLOSIDES EN COSMETIQUE**
VERWENDUNG VON ALKYLPOLYXYLOSIDEN IN KOSMETIKA
USE OF ALKYLPOLYXYLOSIDES IN COSMETICS

(30) Priorité: 05.10.2001 FR 0112821
(43) Date de publication de la demande: 14.07.2004
(73) Titulaire: SOCIETE D'EXPLOITATION DE PRODUITS POUR LES INDUSTRIES CHIMIQUES-SEPPIC, F-75321 Paris Cédex 07 (FR)
(72) Inventeur: AMALRIC, Chantal, F-81700 Blan (FR); ROSO, Alicia, F-81710 Saix (FR); MICHEL, Nelly, F-94700 Maisons-Alfort (FR); TABACCHI, Guy, F-75007 Paris (FR); MILIUS, Alain, F-06000 Nice (FR)
(74) Mandataire: Conan, Philippe Claude
(86) Numéro de dépôt international: PCT/FR2002/003328
(87) Numéro de publication internationale: WO 2003/030861

(56) Documents cités:
- EP-A- 0 507 047
- EP-A- 1 142 901
- DE-A- 4 311 159
- FR-A- 2 756 195
- US-A- 5 362 494
- US-A- 5 681 949
- US-H- H 171

## Description

La présente invention a pour objet l'utilisation d'alkylpolyxylosides particuliers comme agents améliorant le toucher cosmétique d'émulsions huile-dans-eau contenant un polymère, ainsi que les émulsions huile-dans-eau comprenant de tels alkylpolyxylosides.

Les émulsions cosmétiques à phase continue aqueuse (c'est-à-dire de type huile-dans-eau ou H/E) contiennent de plus en plus fréquemment des polymères, utilisés en tant qu'épaississants, émulsionnants ou stabilisants. L'utilisation de ces polymères permet de réduire ou supprimer la quantité d'émulsionnants traditionnels. Cependant le toucher des compositions cosmétiques ainsi obtenues perd en richesse et devient aqueux.

La demande de brevet EP 1 142 901 A1, relève de l'art. 54 (3) CBE et décrit des composés de formule :

R-O-(X)ₚ

dans laquelle :
p représente un nombre décimal compris entre 1 et 5,
X représente le reste du xylose, et
R représente un radical alkyle ramifié de formule:

   CH(CₙH₂ₙ₊₁)(CₘH₂ₘ₊₁)-CH₂-
dans laquelle m est un nombre entier compris entre 6 et 18, n est un nombre entier compris entre 4 et 18 et la somme n + m est supérieure ou égale à 10.

Ces composés sont indiqués comme agents tensioactifs.

II a maintenant été découvert que des alkylpolyxylosides obtenus à partir d'un alcool de Guerbet ayant de 16 à 28 atomes de carbone, permettent d'améliorer le toucher des émulsions huile-dans-eau contenant un polymère.

Ainsi, selon un premier aspect, l'invention a pour objet l'utilisation d'un alkylpolyxyloside de formule :

R-O-(X)ₚ (I)

dans laquelle :
p représente un nombre décimal compris entre 1 et 5,
X représente le reste du xylose, et
R représente un radical alkyle ramifié de formule :

   CH(CₙH₂ₙ₊₁)(CₘH₂ₘ₊₁)-CH₂-

   dans laquelle m est un nombre entier compris entre 6 et 12, n est un nombre entier compris entre 8 et 16 et la somme n + m est comprise dans la gamme de 14 à 26 ;
   ou bien d'une composition consistant en un mélange d'au moins deux alkylpolyxylosides tels que définis ci-dessus ;
   comme agent améliorant le toucher cosmétique d'émulsions eau-dans-huile contenant un ou plusieurs polymères.

De préférence, la somme n + m est égale à 14, 16, 18, 22 ou 26 et R représente plus particulièrement un radical 2-hexyldécyle (m = 6, n = 8), 2-octyldécyle (m = 8, n = 8), 2-hexyldodécyle (m = 6, n = 10), 2-octyldodécyle (m = 8, n = 10), 2-décyltétradécyle (m = 10, n = 12) ou 2-dodécylhexadécyle (m = 12, n = 14). De manière particulièrement préférée, la somme m + n est supérieure à 16, et est avantageusement égale à 18, 22 ou 26, de préférence encore égale à 22 ou 26.

Dans la formule R-O-(X)ₚ, le groupe R-O- est lié à X par le carbone anomérique du reste de xylose, de manière à former une fonction acétal.

p, qui représente le degré moyen de polymérisation du xylose, est plus particulièrement compris entre 1 et 2,5 et tout particulièrement entre 1 et 2,0.

Le composé de formule R-O-(X)ₚ peut être préparé en faisant réagir le xylose avec un excès d'alcool gras de formule ROH, puis élimination de l'alcool gras n'ayant pas réagi.

Dans le procédé tel que défini ci-dessus, la réaction est effectuée en présence de catalyseurs acides forts.

Selon une variante du procédé tel que défini précédemment, le xylose est mis à réagir avec un alcool de formule R₁-OH, dans laquelle R₁ comporte de 1 à 4 atomes de carbone et plus particulièrement, avec le butanol, pour conduire à l'acétal de formule R₁O-(X)ₚ, qui subit ensuite une trans-acétalisation par un excès d'alcool de formule ROH avec distillation de l'alcool de formule R₁OH formé puis élimination de l'alcool de formule ROH n'ayant pas réagi.

Dans ce procédé et sa variante, tels que décrits précédemment, l'élimination de l'alcool de formule ROH n'ayant pas réagi, est effectuée selon des méthodes connues de l'homme du métier comme, par exemple, la distillation, la distillation sur film mince, la distillation moléculaire ou l'extraction par solvants.

Selon un second aspect, la présente invention a pour objet une émulsion huile-dans-eau comprenant :
- de 0,1 à 15 % en poids d'un ou plusieurs alkylpolyxylosides tels que définis ci-dessus ;
- de 0,05 à 10 % en poids d'un ou plusieurs polymères;
- plus de 5 % en poids, de préférence plus de 7 % en poids, et de préférence encore plus de 10 % en poids, et jusqu'à 50 % en poids d'une phase grasse constituée d'une ou plusieurs huiles et/ou d'une ou plusieurs cires.

Selon un mode de réalisation avantageux, l'alkylpolyxyloside de formule (I) est en mélange avec son alcool de Guerbet correspondant (de formule ROH où R a la signification mentionnée précédemment), dans un rapport pondéral alkylpolyxyloside/alcool compris dans la gamme de 1/99 à 99/1.

Parmi les polymères utilisés dans l'émulsion huile-dans-eau selon la présente invention, on citera notamment les homopolymères ou copolymères d'acide acrylique, de dérivés d'acide acrylique, d'acrylamide et de ses dérivés, de l'acide acrylamidométhylpropanesulfonique, de monomère vinylique, de chlorure de triméthylaminoéthylacrylate comme par exemple les produits commercialisés sous la dénomination CARBOPOL® Ultrez 10, PEMULEN® TR1 et TR2, SIMULGEL®A, SIMULGEL ®NS, SIMULGEL®EPG, SIMULGEL®EG, LUVIGEL®EM, SALCARE®SC91, SALCARE®SC92, SALCARE®SC95, SALCARE®SC96, FLOCARE®ET100, HISPAGEL®, SEPIGEL®305, SEPIGEL®501, SEPIGEL®502, FLOCARE®ET58, STABILEZE®06 ; les hydrocolloïdes d'origine végétale ou biosynthétique comme par exemple la gomme de xanthane, la gomme de karaya, les carraghénates, les alginates ; les silicates ; la cellulose et ses dérivés ; l'amidon et ses dérivés hydrophiles ; les polyuréthanes.

Parmi les huiles utilisables dans le cadre de la présente invention, on peut citer notamment :
- les huiles d'origine végétale, telles que l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula ;
- les huiles végétales et leurs esters méthyliques éthoxylés ;
- les huiles d'origine animale, telles que le squalène, le squalane ;
- les huiles minérales, telles que l'huile de paraffine, l'huile de vaseline et les isoparaffines ;
- les huiles synthétiques, notamment les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les polyalphaoléfines, les polyoléfines comme le polyisobutène, les isoalcanes de synthèse comme l'isohexadecane, l'isododécane, les huiles perfluorées et les huiles de silicone.

Parmi les cires utilisables dans le cadre de la présente invention, on peut citer par exemple la cire d'abeille ; la cire de carnauba ; la cire de candelilla ; la cire d'ouricoury ; la cire du Japon ; la cire de fibre de liège ou de canne à sucre ; les cires de paraffines ; les cires de lignite ; les cires microcristallines ; la cire de lanoline ; l'ozokérite ; la cire de polyéthylène ; les huiles hydrogénées ; les cires de silicone ; les cires végétales ; les alcools gras et les acides gras solides à température ambiante ; les glycérides solides à température ambiante.

L'émulsion eau-dans-huile conforme à la présente invention peut également contenir, de façon optionnelle, jusqu'à 15 % en poids d'un émulsionnant.

Parmi les émulsionnants utilisables dans le cadre de la présente invention, on peut citer par exemple les acides gras ; les acides gras éthoxylés ; les esters d'acide gras et de sorbitol ; les esters d'acides gras éthoxylés ; les polysorbates ; les esters de polyglycérol ; les alcools gras éthoxylés ; les esters de sucrose ; les alkylpolyglycosides ; les alcools gras sulfatés et phosphatés ;

D'une façon connue en soi, ces émulsions peuvent en outre comprendre un ou plusieurs composés choisis parmi les humectants, comme par exemple la glycérine, les conservateurs, les colorants, les parfums, les actifs cosmétiques, les filtres solaires minéraux ou organiques, les charges minérales comme les oxydes de fer, oxydes de titane et le talc, les charges synthétiques comme les nylons et les poly(méthacrylate de méthyle) réticulés ou non, les élastomères silicone, les séricites et les extraits de plantes.

Les émulsions huile-dans-eau conformes à l'invention peuvent être préparées de la manière suivante.

La phase aqueuse est chauffée à une température de 70 à 85°C. Parallèlement la phase grasse contenant le système émulsionnant de l'invention et les huiles (éventuellement additivée par des cires, des coémulsionnants et des actifs lipophiles) est chauffée à une température identique de 70 à 85°C. Les deux phases sont ensuite mélangées et émulsionnées à l'aide d'un émulseur de type rotor stator (par exemple un mélangeur de laboratoire SILVERSON). Après quelques minutes d'émulsification, l'émulsion est refroidie sous agitation modérée. Le ou les polymères sont introduits dans la phase huileuse, dans la phase aqueuse ou directement dans l'émulsion selon les recommandations des fournisseurs.

Si tous les composants de l'émulsion sont liquides, la fabrication peut être réalisée sans chauffage.

L'invention sera illustrée par les exemples suivants.

### Exemple 1 : Préparation d'un 2-décyltétradécylxyloside

61,8 kg de 2-décyltétradécanol, commercialisé par la Société SASOL sous l'appellation Isofol®24, sont introduits dans un réacteur. 8,7 kg de xylose sont dispersés progressivement dans le milieu agité et 65 g d'acide sulfurique sont alors ajoutés. Le mélange est maintenu à 115°C pendant 6 heures, sous vide partiel, puis neutralisé par la lessive de soude. Après filtration, le liquide limpide obtenu présente un indice d'hydroxyle de 183 et contient 15% en poids de 2-décyltétradécylxyloside et 85% en poids de 2-décyltétradécanol.

### Exemple 2: Mise en évidence de l'amélioration du toucher obtenue lorsque l'on ajoute le composé selon l'exemple 1 dans des préparations cosmétiques ne contenant pas d'émulsionnant.

On prépare 2 séries d'émulsions ayant les compositions suivantes :

| | |
|---|---|
| 1ère série | |
| Octanoate de cétéaryle | 10% |
| Eau | qsp 100% |
| Polymère | qs |
| Conservateurs | qs |
| 2^{ème} série | |
| Composé selon l'exemple 1 | 3% |
| Octanoate de cétéaryle | 10% |
| Eau | qsp 100% |
| Polymère | qs |
| Conservateurs | qs |

Mode de préparation : le polymère est dispersé dans l'eau et neutralisé si nécessaire, puis l'octanoate de cétéaryle est introduit dans le gel formé. L'homogénéisation de l'émulsion est réalisée sans chauffage par agitation avec les équipements traditionnels. Le composé selon l'exemple 1 est ajouté avec l'octanoate de cétéaryle.

### Les préparations obtenues sont des émulsions H/E.

Une évaluation sensorielle est réalisée sur un panel de 20 volontaires entraînés. Les résultats sont présentés dans le Tableau 1. Chaque critère est noté de 0 à 5.

**TABLEAU 1**

| Polymère | Polyacrylamide et C11-13 isoparaffin et laureth-7 : 3% | Carbomer 0.4% et acrylates/steareth-20 méthacrylate copolymère 0.2% | Acryloyl dimethyl taurate copolymère 1 % |
|---|---|---|---|
| Evaluation des émulsions sans le composé selon l'exemple 1 | | | |
| Richesse | 2 | 0 | 0 |
| Film résiduel | 2 | 1 | 1 |
| Evaluation des émulsions contenant 3% du composé selon l'exemple 1 | | | |
| Richesse | 3 | 2 | 3 |
| Film résiduel | 4 | 3 | 3 |

L'ajout de 3% du composé selon l'exemple 1 dans les émulsions à base de polymère améliore significativement la richesse et augmente la sensation d'émollience en laissant un film résiduel significativement perçu par les volontaires.

### Exemple 3 : Mise en évidence de l'amélioration du toucher obtenue lorsque l'on ajoute le composé selon l'exemple 1 dans des préparations cosmétiques contenant un émulsionnant

On prépare 2 émulsions ayant la composition suivante :

| | | |
|---|---|---|
| Emulsionnant | 3% | 3% |
| Composé selon l'exemple 1 | | 3% |
| Octanoate de cétéaryle | 10% | 10% |
| Eau | qsp 100% | qsp 100% |
| Polyacrylamide et C11-13 Isoparaffin et Laureth-7 | 1.5% | 1.5% |
| Conservateurs | qs | qs |

La préparation de ces émulsions est réalisée selon un procédé classique par émulsification à chaud des 2 phases aqueuses et huileuses.

Les préparations obtenues sont des émulsions H/E.

Une évaluation sensorielle est réalisée sur un panel de 20 volontaires entraînés. Les résultats sont présentés dans le Tableau 2. Chaque critère est noté de 0 à 5.

**TABLEAU 2**

| Emulsionnant | Arachidyl alcohol et Behenyl alcohol et Arachidyl glucoside | PEG100 steareate et glyceryl stearate | Ceteth2 + ceteth 21 |
|---|---|---|---|
| Evaluation des essais sans le composé selon l'exemple 1 | | | |
| Richesse | 1 | 1 | 0 |
| Film résiduel | 1 | 1 | 1 |
| Evaluation des essais contenant 3% du composé selon l'exemple 1 | | | |
| Richesse | 3 | 2 | 2 |
| Film résiduel | 3 | 3 | 3 |

L'ajout de 3% du composé selon l'exemple 1 permet d'améliorer significativement le profil sensoriel des émulsions testées en augmentant simultanément la richesse et le film résiduel.

## Revendications

1. Utilisation d'un alkylpolyxyloside ou d'un mélange d'alkylpolyxylosides de formule :
R-O-(X)ₚ (I)
dans laquelle :
p représente un nombre décimal compris entre 1 et 5,
X représente le reste du xylose, et
R représente un radical alkyle ramifié de formule :
CH(CₙH₂ₙ₊₁) (CₘH₂ₘ₊₁)-CH₂-
dans laquelle m est un nombre entier compris entre 6 et 12, n est un nombre entier compris entre 8 et 16 et la somme n + m est comprise dans la gamme de 14 à 26 ;
comme agent améliorant le toucher cosmétique d'émulsions huile-dans-eau contenant un ou plusieurs polymères.

2. Utilisation selon la revendication 1, dans laquelle dans la formule (I) la somme m + n est égale à 14, 16, 18, 22 ou 26, et est de préférence supérieure à 16 et encore de préférence égale à 18, 22 ou 26.

3. Utilisation selon la revendication 1, dans laquelle dans la formule (I) la somme m + n est égale à 22 ou 26.

4. Utilisation selon la revendication 1, 2 ou 3, dans laquelle dans la formule (I) R représente un radical 2-hexyldécyle, 2-octyldécyle, 2-hexyldodécyle, 2-octyldodécyle, 2-décyltétradécyle ou 2-dodécylhexadécyle.

5. Emulsion huile-dans-eau, comprenant :
- de 0,1 à 15 % en poids d'un alkylpolyxyloside ou d'un mélange d'alkylpolyxylosides de formule :
R-O-(X)ₚ (I)
dans laquelle :
p représente un nombre décimal compris entre 1 et 5,
X représente le reste du xylose, et
R représente un radical alkyle ramifié de formule :
CH(CₙH₂ₙ₊₁)(CₘH₂ₘ₊₁)-CH₂-
dans laquelle m est un nombre entier compris entre 6 et 12, n est un nombre entier compris entre 8 et 16 et la somme n + m est comprise dans la gamme de 14 à 26 ;
- de 0,05 à 10 % en poids d'un ou plusieurs polymères ; et
- plus de 5 % en poids et jusqu'à 50 % en poids d'une phase grasse constituée d'une ou plusieurs huiles et/ou d'une ou plusieurs cires.

6. Emulsion selon la revendication 5, dans laquelle chaque alkylpolyxyloside est en mélange avec son alcool correspondant de formule ROH, dans un rapport pondéral alkylpolyxyloside/alcool compris dans la gamme de 1/99 à 99/1.

7. Emulsion selon la revendication 5 ou 6, qui comprend également jusqu'à 15 % en poids d'un émulsionnant.

8. Emulsion selon l'une des revendications 5 à 7, dans laquelle dans la formule (I) la somme m + n est égale à 14, 16, 18, 22 ou 26, et est de préférence supérieure à 16 encore de préférence à 16, 22 ou 26.

9. Emulsion selon l'une des revendications 5 à 7, dans laquelle dans la formule (I) la somme m + n est égale à 22 ou 26.

10. Emulsion selon l'une des revendications 5 à 9, dans laquelle dans la formule (I) R représente un radical 2-hexyldécyle, 2-octyldécyle, 2-hexyldodécyle, 2-octyldodécyle, 2-décyltétradécyle ou 2-dodécylhexadécyle.

## Claims

1. Use of an alkylpolyxyloside or of a mixture of alkylpolyxylosides of formula:
R-O- (X)_{P} (I)
in which:
p represents a decimal number between 1 and 5,
X represents a xylose residue, and
R represents a branched alkyl radical of formula:
CH ( CₙH₂ₙ₊₁) (CₘH₂ₘ₊₁) - CH₂ -
in which m is an integer between 6 and 12, n is an integer between 8 and 16 and the sum n + m is in the range from 14 to 26;
as agent enhancing the cosmetic feel of oil-in-water emulsions containing one or more polymers.

2. Use according to Claim 1, in which in formula (I) the sum m + n is equal to 14, 16, 18, 22 or 26, and is preferably greater than 16 and is more preferably still equal to 18, 22 or 26.

3. Use according to Claim 1, in which in formula (I) the sum m + n is equal to 22 or 26.

4. Use according to Claim 1, 2 or 3, in which in formula (I) R represents a 2-hexyldecyl, 2-octyldecyl, 2-hexyldodecyl, 2-octyldodecyl, 2-decyltetradecyl or 2-dodecylhexadecyl radical.

5. Oil-in-water emulsion comprising:
- from 0.1 to 15% by weight of an alkylpolyxyloside or of a mixture of alkylpolyxylosides of formula:
R-O- (X)ₚ (I)
in which:
p represents a decimal number between 1 and 5,
X represents a xylose residue, and
R represents a branched alkyl radical of formula:
CH (CₙH₂ₙ₊₁) (CₘH₂ₘ₊₁) - CH2 -
in which m is an integer between 6 and 12, n is an integer between 8 and 16 and the sum n + m is in the range from 14 to 26;
- from 0.05 to 10% by weight of one or more polymers; and
- more than 5% by weight and up to 50% by weight of a fatty phase consisting of one or more oils and/or one or more waxes.

6. Emulsion according to Claim 5, in which each alkylpolyxyloside is in the form of a mixture with its corresponding alcohol of formula ROH, in an alkylpolyxyloside/alcohol weight ratio in the range from 1/99 to 99/1.

7. Emulsion according to Claim 5 or 6, which also comprises up to 15% by weight of an emulsifier.

8. Emulsion according to one of Claims 5 to 7, in which in formula (I) the sum m + n is equal to 14, 16, 18, 22 or 26, and is preferably greater than 16, more preferably still than 16, 22 or 26.

9. Emulsion according to one of Claims 5 to 7, in which in formula (I) the sum m + n is equal to 22 or 26.

10. Emulsion according to one of Claims 5 to 9, in which in formula (I) R represents a 2-hexyldecyl, 2-octyldecyl, 2-hexyldodecyl, 2-octyldodecyl, 2-decyltetradecyl or 2-dodecylhexadecyl radical.

## Patentansprüche

1. Verwendung eines Alkylpolyxylosids oder von einer Mischung von Alkylpolyxylosiden der Formel:
R-O-(X)ₚ (I)
in der
p eine Dezimalzahl zwischen 1 und 5 bedeutet,
X den Xyloserest bedeutet, und
R einen verzweigten Alkylrest der Formel:
CH (CₙH₂ₙ₊₁) (CₘH₂ₘ₊₁)-CH₂-
bedeutet, wobei m eine ganze Zahl zwischen 6 und 12 bedeutet, n eine ganze Zahl zwischen 8 und 16 bedeutet und die Summe n + m im Bereich von 14 bis 26 liegt;
als Mittel, das das kosmetische Gefühl von Öl-in-Wasser-Emulsionen, die einen oder mehrere Polymere enthalten, verbessert.

2. Verwendung nach Anspruch 1, wobei in Formel (I) die Summe m + n gleich 14, 16, 18, 22 oder 26 beträgt und vorzugsweise größer als 16 ist und noch stärker bevorzugt gleich 18, 22 oder 26 ist.

3. Verwendung nach Anspruch 1, wobei in Formel (I) die Summe m + n gleich 22 oder 26 ist.

4. Verwendung nach Anspruch 1, 2 oder 3, wobei in Formel (I) R einen 2-Hexyldecyl-, 2-Octyldecyl-, 2-Hexyldodecyl-, 2-Octyldodecyl-, 2-Decyltetradecyl- oder 2-Dodecylhexadecylrest bedeutet.

5. Öl-in-Wasser-Emulsion, die Folgendes umfasst:
- 0,1 bis 15 Gew.-% an einem Alkylpolyxylosid oder an einer Mischung von Alkylpolyxylosiden der Formel:
R-O-(X)ₚ (I)
in der
p eine Dezimalzahl zwischen 1 und 5 bedeutet,
X den Xyloserest bedeutet, und
R einen verzweigten Alkylrest der Formel:
CH (CₙH₂ₙ₊₁) (CₘH₂ₘ₊₁)-CH₂-
bedeutet, wobei m eine ganze Zahl zwischen 6 und 12 bedeutet, n eine ganze Zahl zwischen 8 und 16 bedeutet und die Summe n + m im Bereich von 14 bis 26 liegt;
- 0,05 bis 10 Gew.-% an einem oder mehreren Polymeren; und
- mehr als 5 Gew.-% und bis zu 50 Gew.-% an einer Fettphase, die aus einem oder mehreren Ölen und/oder einem oder mehreren Wachsen besteht.

6. Emulsion nach Anspruch 5, in der jedes Alkylpolyxylosid in Abmischung mit seinem entsprechenden Alkohol der Formel ROH in einem Gewichtsverhältnis Alkylpolyxylosid/Alkohol im Bereich von 1/99 bis 99/1 vorliegt.

7. Emulsion nach Anspruch 5 oder 6, die bis zu 15 Gew.-% an einem Emulgator umfasst.

8. Emulsion nach einem der Ansprüche 5 bis 7, wobei in Formel (I) die Summe m + n gleich 14, 16, 18, 22 oder 26 beträgt und vorzugsweise größer als 16 ist und noch stärker bevorzugt gleich 16, 22 oder 26 ist.

9. Emulsion nach einem der Ansprüche 5 bis 7, wobei in Formel (I) die Summe m + n gleich 22 oder 26 ist.

10. Emulsion nach einem der Ansprüche 5 bis 9, wobei in Formel (I) R einen 2-Hexyldecyl-, 2-Octyldecyl-, 2-Hexyldodecyl-, 2-Octyldodecyl-, 2-Decyltetradecyl- oder 2-Dodecylhexadecylrest bedeutet.
